(19) Europäisches Patentamt / European Patent Office / Office européen des brevets

(11) **EP 2 354 990 A1**

(12) **EUROPEAN PATENT APPLICATION**

(43) Date of publication:
**10.08.2011 Bulletin 2011/32**

(51) Int Cl.:
*G06F 19/00 (2011.01)*

(21) Application number: **10152574.9**

(22) Date of filing: **03.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
- **Alferness, Clifton A.**
  **Port Orchard, WA 98367 (US)**
- **Bardy, Gust H.**
  **Redmond, WA 98053 (US)**

(72) Inventors:
- **Alferness, Clifton A.**
  **Port Orchard, WA 98367 (US)**
- **Bardy, Gust H.**
  **Redmond, WA 98053 (US)**

(74) Representative: **Curley, Donnacha John et al**
**Hanna Moore & Curley**
**13 Lower Lad Lane**
**Dublin 2 (IE)**

# (54) System and method for actively managing type 1 diabetes mellitus on a personalized basis

(57) A system (140) and method (30) for actively managing Type 1 diabetes mellitus on a personalized basis is provided. Models of glycemic effect for a Type 1 diabetic patient (21) are established for both insulin time course (50) and digestive response (40). A rise (157) in postprandial blood glucose is estimated through food ingestion of a planned meal (71) in proportion to the digestive response model (40). An amount of insulin (158) necessary and timing of delivery (156) to mediate transport of blood glucose into cells in proportion to the postprandial blood glucose rise (157) is determined through the insulin time course model (51).

**Fig. 6.**

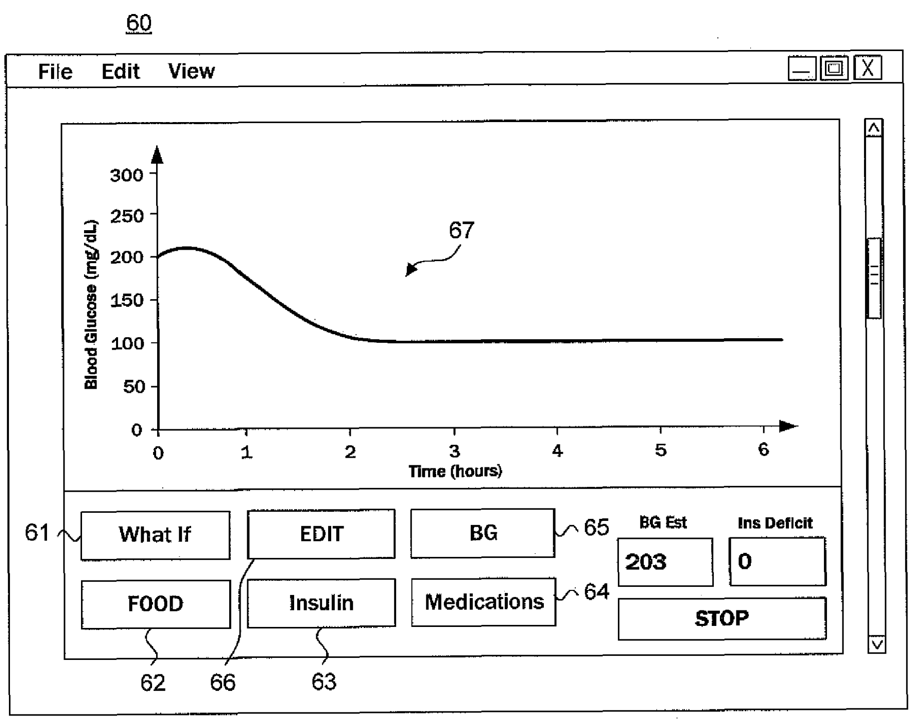


EP 2 354 990 A1

## Description

### Field

This application relates in general to Type 1 diabetes mellitus management and, in particular, to a system and method for actively managing Type 1 diabetes mellitus on a personalized basis.

### Background

Diabetes mellitus, or simply, "diabetes," is an incurable chronic disease. Type 1 diabetes is caused by the destruction of pancreatic beta cells in the Islets of Langerhans through autoimmune attack. Type 2 diabetes is due to defective insulin secretion, insulin resistance, or reduced insulin sensitivity. Gestational diabetes first appears during pregnancy and generally resolves after childbirth, absent preexisting weak pancreatic function. Less common forms of diabetes include thiazide-induced diabetes, and diabetes caused by chronic pancreatitis, tumors, hemochromatosis, steroids, Cushing's disease, and acromegaly.

Type 1 diabetics must manage their diabetes by taking insulin to compensate for the rise in blood glucose that follows food consumption. Type 1 diabetes management works to prevent hyperglycemia, or high blood glucose, while especially averting the consequences of hypoglycemia, or low blood glucose, from over-aggressive or incorrect insulin dosing. Poor diabetes management can manifest in acute symptoms, such as loss of consciousness, or through chronic conditions, including cardiovascular disease, retinopathy, neuropathy, and nephropathy.

Type 1 diabetics often develop an intuition over their own insulin sensitivity and learn to counterbalance the effects of an insulin dosing regimen through control over diet and exercise. For instance, adhering to a diet with a moderate level of carbohydrates and regularly performing blood glucose self-testing help to control lability or brittleness.

Effective diabetes management requires effort. Inexperience, lack of self discipline, and indifference can result in poor diabetes management. Intuition is not infallible and well-intentioned insulin dosing is of little use if the patient forgets to actually take his insulin or disregards dietary planning. Similarly, a deviation from dietary planning followed by a remedial insulin dosage can result in undesirable blood glucose oscillations. Physiological factors, well beyond the value of intuition or skill, such as illness, stress, and general well-being, can also complicate management.

Despite the importance of effective management, Type 1 diabetics seldom receive direct day-to-day oversight. Physician experience, patient rapport, and constrained clinic time pose limits on the amount and quality of oversight provided. Physicians are often removed in time and circumstance from significant metabolic events and minor blood glucose aberrations and often wide fluctuations may not present in-clinic when a physician can actually observe them. Primary care and endocrinologist visits occur infrequently and at best provide only a "snapshot" of diabetes control. For instance, glycated hemoglobin (HbA1c) is tested every three to six months to evaluate long-term control, yet reflects a bias over more recent blood glucose levels.

Existing approaches to diabetes management still rely on physician decision-making. For instance, U.S. Patent No. 6,168,563, to Brown, discloses a healthcare maintenance system based on a hand-held device. Healthcare data, such as blood glucose, can be uploaded on to a program cartridge for healthcare professional analysis at a centralized location. Healthcare data can also be directly obtained from external monitoring devices, including blood glucose monitors. At the centralized location, blood glucose test results can be matched with quantitative information on medication, meals, or other factors, such as exercise. Changes in medication dosage or modification to the patient's monitoring schedule can be electronically sent back to the patient. However, decision making on day-to-day diabetes management through interpretation of uploaded healthcare data remains an offline process, being discretionary to the remote healthcare professional and within his sole control and timing.

Similarly, U.S. Patent No. 6,024,699, to Surwit et al. ("Surwit"), discloses monitoring, diagnosing, prioritizing, and treating medical conditions of a plurality of remotely located patients. Each patient uses a patient monitoring system that includes medicine dosage algorithms, which use stored patient data to generate dosage recommendations for the patient. A physician can modify the medicine dosage algorithms, medicine doses, and fixed or contingent self-monitoring schedules, including blood glucose monitoring through a central data processing system. Diabetes patients can upload their data to the central data processing system, which will detect any trends or problems. If a problem is detected, a revised insulin dosing algorithm, insulin dosage, or self-monitoring schedule can be downloaded to their patient monitoring system. However, any changes to diabetes management remain within the sole discretion and timing of a physician, who acts remotely in place and time via the central data processing system.

Therefore, there is a need for an approach to Type 1 diabetes management with provisions for customizing insulin and dietary parameters to meet a diabetic's personal sensitivities and day-to-day needs without the delay inherent in current diabetes management.

## Summary

A system and method for interactively managing Type 1 diabetes on an individualized and patient-specific basis is provided for use at any time and in any place and for any diet under any metabolic circumstance. Models of glycemic effect by insulin, by antidiabetic and oral medications, if applicable, and by food consumption are formed based on sensitivities particular to a diabetic patient. A rise in blood glucose is estimated based on food selections indicated by the patient, which is adjusted to compensate for the patient's specific carbohydrate sensitivity, as well as for any supervening physiological or pathophysiological influences. Similarly, an amount of insulin necessary to counteract the rise in blood glucose over the expected time course is determined, also adjusted to match the patient's insulin sensitivity. The antidiabetic and oral medications are similarly considered in light of glycemic effect, if appropriate.

One embodiment provides a system and method for actively managing Type 1 diabetes mellitus on a personalized basis. Models of glycemic effect for a Type 1 diabetic patient are established for both insulin time course and digestive response. A rise in postprandial blood glucose is estimated through food ingestion of a planned meal in proportion to the digestive response model. An amount of insulin necessary and timing of delivery to mediate transport of blood glucose into cells in proportion to the postprandial blood glucose rise is determined through the insulin time course model.

A further embodiment provides a system and method for managing Type 1 diabetes mellitus through a personal predictive management tool. A personal insulin response profile is referenced for a patient of Type 1 diabetes mellitus for a type of insulin preparation. A time course curve for a patient population is maintained and includes expected blood glucose levels for a type of human-consumable food. The blood glucose levels following consumption of the food are estimated by evaluating an interaction between the personal insulin response profile and the time course curve over a duration of action of the insulin preparation.

The personal predictive management tool provides Type 1 diabetics with a new-found sense of personal freedom and safety by integrating the vagaries of daily blood glucose control into a holistic representation that can be applied and refined to keep pace with the unpredictable nature of daily life. The approach described herein closely approximates what a normal pancreas does by interactively guiding the individual diabetic under consideration and, over time, learning how the patient can be understood and advised.

This approach also extends beyond the prevention of hyperglycemia and includes the prevention of hypoglycemia. Hypoglycemic episodes are a bane to insulin users and can result in confusion, syncope, seizures, falls, automobile accidents, and embarrassment, all of which result from the confusing mental state that results when blood glucose falls below 65 or thereabouts in most people. As a matter of practical day-to-day diabetes management, hypoglycemia is more of a concern to the insulin user than the long term consequences of hyperglycemia. The negative consequences of hyperglycemia seem remote to most patients who fear the immediate negative consequence of hypoglycemia in any of the traditional approaches to strictly control their blood glucose. Thus, the concern over hypoglycemic symptoms often prevents patients from optimally controlling their blood glucose levels. The approach provided herein takes into account the problem of hypoglycemia with the same rigor as that applied to hyperglycemia.

Still other embodiments of the present invention will become readily apparent to those skilled in the art from the following detailed description, wherein are described embodiments by way of illustrating the best mode contemplated for carrying out the invention. As will be realized, the invention is capable of other and different embodiments and its several details are capable of modifications in various obvious respects, all without departing from the spirit and the scope of the present invention. Accordingly, the drawings and detailed description are to be regarded as illustrative in nature and not as restrictive.

## Brief Description of the Drawings

FIGURE 1 is a functional block diagram showing, by way of example, a prior art diabetes management cycle for a typical Type 1 diabetic.

FIGURE 2 is a functional block diagram showing, by way of example, an automated diabetes management cycle for a typical Type 1 diabetic, in accordance with one embodiment.

FIGURE 3 is a process flow diagram showing personalized Type 1 diabetes mellitus modeling.

FIGURE 4 is a graph showing, by way of example, a digestive response curve for a standardized test meal.

FIGURE 5 is a graph showing, by way of example, an insulin activity curve for lispro, an insulin analog.

FIGURE 6 is a diagram showing, by way of example, a screen shot of a graphical user interface for performing automated management of Type 1 diabetes, in accordance with one embodiment.

FIGURE 7 is a diagram showing, by way of example, a screen shot of a graphical user interface for selecting food combinations for use in the graphical user interface of FIGURE 6.

FIGURES 8A-C are graphs showing, by way of example, constituent and cumulative digestive response curves for a hypothetical meal.

FIGURE 9 is a diagram showing, by way of example, a screen shot of a graphical user interface for specifying insulin preparation type for use in the graphical user interface of FIGURE 6.

FIGURE 10 is a diagram showing, by way of example, a screen shot of a graphical user interface for specifying other medications for use in the graphical user interface of FIGURE 6.

FIGURE 11 is a process flow diagram showing a method for actively managing Type 1 diabetes mellitus on a personalized basis, in accordance with one embodiment.

FIGURE 12 is a process flow diagram showing a routine for refining a food data library for use with the method of FIGURE 11.

FIGURE 13 is a process flow diagram showing a routine for interacting with a patient for use with the method of FIGURE 11.

FIGURE 14 is a block diagram showing for a system for actively managing Type 1 diabetes mellitus on a personalized basis, in accordance with one embodiment.

## Detailed Description

### Diabetes Management Cycles

The principal cause of Type 1 diabetes is a T-cell mediated autoimmune attack on the beta cells of the Islets of Langerhans of the pancreas. No known preventative measures exist. Effective management of Type 1 diabetes requires continual daily control over blood glucose. FIGURE 1 is a functional block diagram showing, by way of example, a prior art diabetes management cycle 10 for a typical Type 1 diabetic. Type 1 diabetes management fundamentally centers on the timing and content of meals, including beverages, and the timing and dosing of insulin, although other factors, such as physical activity and exercise, patient well-being, illness, and stress, can influence the course of management

Consequently, Type 1 diabetes can only be treated through insulin therapy, which is normally combined with adjustments to patient lifestyle, including diet and exercise. As a result, a typical Type 1 diabetic patient 11 learns to plan and time his daily meals (step 12) to estimate an expected rise in blood glucose and to determine appropriate doses of insulin to counteract the expected rise. Conventional dietary planning relies heavily on manual use of exchange lists and carbohydrate counting. A postprandial rise in blood glucose is normal and insulin is generally self-administered prior to eating (step 13). Ideally, a Type 1 diabetic's average blood glucose level should be in the range of 80-120 milligrams per deciliter (mg/dL), although a range of 140-150 mg/dL is often used to prevent potentially life-threatening hypoglycemic events. When properly dosed, the insulin will return blood glucose to a normal range within two to four hours of consuming a meal (step 14).

Physicians encourage each Type 1 diabetic to regularly self-test his blood glucose (step 15) to enable better compensation for patient-specific sensitivities to food and insulin. Self testing results are tracked through a patient log. To self-test, the patient 11 places a drop of blood on a test strip coated with a glucose oxidase or hexokinase enzyme, which is read by a glucose monitor. Blood glucose is normally tested at least daily, although stricter control regimens may require more frequent testing, such as after meals, at bedtime, upon awakening, and at other times. The management cycle (operations 12-15) is repeated over every meal.

Patient logs document the interaction of food, insulin dosing, other medications, if applicable, and patient sensitivities. However, descriptions of food consumed and manner of preparation, precise times between insulin dosing and completion of a meal, and physiological factors, such as mood or wellness, are generally omitted. Further, physician review normally only occurs during clinic visits, or as necessary, but detailed study is infrequent due to the time, effort, and cost of reviewing every Type 1 diabetic patient.

The accuracy and timeliness of a Type 1 diabetes management regimen can be improved by automating day-to-day managerial aspects, which are historically performed through intuition and sporadic re-evaluation. FIGURE 2 is a functional block diagram showing, by way of example, an automated diabetes management cycle 20 for a typical Type 1 diabetic, in accordance with one embodiment. Automation is introduced to move the management cycle significantly closer to a closed loop arrangement. Control is streamlined and steps that remain to be performed by a patient manually are minimized, or even eliminated, which makes such steps less apt to be forgotten or missed.

An automated diabetes management tool applies heuristics to model and calibrate a personalized diabetes control regimen (step 22), as further described below beginning with reference to FIGURE 3. Through the management tool, the patient 21 can plan and time insulin dosing and meals (steps 23 and 25, respectively) more accurately than possible through conventional means, including exchange lists and carbohydrate counting. Dynamically tracked blood glucose predictions (step 24) can also be provided, and self-testing results (step 26) can be provided directly into the management tool for time-responsive integration and evaluation. In a further embodiment, emergent glucose self-testing, such as interstitial glucose testing, supplements manual self-testing or, where sufficiently reliable, replaces manual self-testing to achieve a fully closed loop system when combined with insulin pump therapy. Other management tool aspects are possible.

Automated Management of Type 1 Diabetes

A diabetic patient is himself the best resource available to manage his diabetes. Meals, insulin dosing, and changes in personal well being, as well as departures from such plans, are best known to the patient, who alone is ultimately responsible for adherence to a management regimen. FIGURE 3 is a process flow diagram showing personalized Type 1 diabetes mellitus modeling 30. The method is performed as a series of process steps or operations executed by one or more patient-operable devices, including personal computers, personal digital assistants, programmable mobile telephones, intelligent digital media players, or other programmable devices, that are working individually or collaboratively to apply a personal predictive management tool.

Modeling involves projecting the glycemic effect of planned meals in light of insulin dosing, as well as any other medications, if applicable. Meal planning is particularly important, where the content and timing of meals greatly impacts blood glucose and must be closely controlled by dosed insulin to compensate for the lack of naturally-produced insulin. The management tool performs dietary planning (step 31), which involves determining the glycemic effect of food based on a standardized meal. In a further embodiment, planning also includes projecting the affect of exercise or physical activities that are likely to require appreciable caloric expenditure. Other planning aspects are possible.

Once each planned meal is known, the management tool can model the time courses and amplitudes of change for the meal, dosed insulin, and other non-insulin medications (step 32). Additionally, the management tool can be calibrated as necessary to adjust for self-testing and data recorded by the patient (step 33) through predictive modeling and calibration, such as described in commonly-assigned U.S. Patent application, entitled "System And Method For Creating A Personalized Tool Predicting A Time Course Of Blood Glucose Affect In Diabetes Mellitus," Serial No._________ , pending, and U.S. Patent application, entitled "System And Method For Generating A Personalized Diabetes Management Tool For Diabetes Mellitus," Serial No._________ , pending, the disclosure of which is incorporated by reference. Personalized models of blood glucose affect for insulin time course, the time courses of other medications, and digestive response are established. The models predict the timing and rise or fall of the patient's blood glucose in response to insulin, other medications, and food. Other modeling and calibrations are possible.

Digestive Response and Insulin Activity Curves

Despite many decades of experience, blood glucose management still involves an educated guess at proper insulin dosing, as the content and timing of meals, dosing and timing of insulin, and patient-specific sensitivities can cause departure from expected blood glucose control directions. For instance, the digestive response of each patient's body to food consumption is unique. However, the digestive response characteristics can be normalized through consumption of a standardized test meal, such as a specific number of oat wafers, manufactured, for instance, by Ceapro Inc., Edmonton, Canada, or similar calibrated consumable. FIGURE 4 is a graph 40 showing, by way of example, a digestive response curve 41 for a standardized test meal. The x-axis represents time in minutes and the y-axis represents a cumulative rise of blood glucose measured in milligrams per deciliter (mg/dL). The amplitude of the curve 41 is patient-dependent, as is the timing of the rise. The test meal contains a known quantity of carbohydrate with a specific glycemic index. Thus, the curve 41 can be adapted to other types of foods to estimate glycemic effect and counteraction by insulin dosing. Other models of digestive response are possible.

Similarly, insulin response is dependent upon patient-specific sensitivities, which affect the time of onset, peak time, and duration of action of therapeutic effect. FIGURE 5 is a graph 50 showing, by way of example, a personal insulin activity curve. The *x*-axis represents time in minutes and the y-axis represents incremental blood glucose decrease measured in mg/dL. The personal insulin activity model can be depicted through an approximation of population-based insulin activity curves published by insulin manufacturers and other authoritative sources. The patient-specific insulin activity curve 51 mimics the shape of the population-based insulin activity curves through a curvilinear ramp 52 formed to peak activity time, followed by an exponential decay $\tau$ 53. Thus, for a patient insulin sensitivity coefficient of 90%, a patient-specific insulin activity curve 51 would reflect a ten percent decrease in insulin sensitivity over corresponding population-based insulin activity curve results. Other models of insulin activity are possible.

Graphical User Interface

Personalized Type 1 diabetes mellitus management can be provided through a patient-operable interface through which glycemic effect prediction and patient interaction can be performed. FIGURE 6 is a diagram showing, by way of example, a screen shot of a graphical user interface 60 for performing automated management of Type 1 diabetes, in accordance with one embodiment. The user interface 60 provides logical controls that accept patient inputs and display elements that present information to the patient. The logical controls include buttons, or other forms of option selection, to access further screens and menus to estimate glucose rise and insulin needed to counteract the rise 61 ("What If"); plan meals 62 ("FOOD"), as further described below with reference to FIGURE 7; specify an insulin bolus 63 ("Insulin"), as further described below with reference to FIGURE 9; specify other antidiabetic and oral medications 64 ("Medications"), as further described below with reference to FIGURE 10; enter a measured blood glucose reading 65 ("BG"); and edit information 66 ("EDIT"). Further logical control and display elements are possible.

To assist the patient with planning, a graphical display provides a forecast curve 67, which predicts combined insulin dosing, antidiabetic and oral medication administration, and postprandial blood glucose. The *x*-axis represents time in hours and the *y*-axis represents the blood glucose level measured in mg/dL. Modeling estimates the timing and amplitude of change in the patient's blood glucose in response to the introduction of a substance, whether food, physiological state, or drug, that triggers a physiological effect in blood glucose. Generally, actions, such as insulin dosing, medication administration, exercise, and food consumption cause a measureable physiological effect, although other substances and events can influence blood glucose. The time courses and amplitudes of change are adjusted, as appropriate, to compensate for patient-specific factors, such as level of sensitivity or resistance to insulin, insulin secretion impairment, carbohydrate sensitivity, and physiological reaction to medications. In a further embodiment, the management tool includes a forecaster that can identify a point at which an expected blood glucose level from the personal insulin response profile is expected to either exceed or fall below a blood glucose level threshold, which respectively corresponds to hypoglycemia and hyperglycemia. Other actions and patient-specific factors, like exercise or supervening illness, may also alter the time courses and amplitudes of blood glucose.

In one embodiment, a meal is planned through a food selection user interface, as further described below with reference to FIGURE 7, and insulin dosing is estimated through an insulin selection user interface, as further described below with reference to FIGURE 9. The digestive response, insulin, and any other medication activity curves are combined, so the effect of the insulin dosing and drugs, if applicable, can be weighed against the proposed meal. Other forecasting aids are possible.

In one embodiment, the user interface 60 and related components are implemented using a data flow programming language provided through the LabVIEW development platform and environment, licensed by National Instruments Corporation, Austin, TX although other types and forms of programming languages, including functional languages, could be employed. The specific option menus will now be discussed.

## Food Selection

Estimating postprandial glucose rise involves modeling food constituents as combined into a meal of specific food types, portion sizes, and preparations. FIGURE 7 is a diagram showing, by way of example, a screen shot of a graphical user interface 70 for selecting food combinations for use in the graphical user interface 60 of FIGURE 6. Dietary management, and thence, the management tool, focuses on carbohydrates, which have the greatest affect on blood glucose. Simple sugars increase blood glucose rapidly, while complex carbohydrates, such as whole grain bread, increase blood glucose more slowly due to the time necessary to break down constituent components. Fats, whether triglyceride or cholesterol, neither raise nor lower blood glucose, but can have an indirect affect by delaying glucose uptake. Proteins are broken down into amino acids, which are then converted into glucose that will raise blood glucose levels slowly. Proteins will not generally cause a rapid blood glucose rise. Nevertheless, both fats and proteins are incorporated into the model by virtue of their empiric effect on blood glucose levels. Additionally, various combinations or preparations of medications or food can have synergistic effects that can alter blood glucose rise and timing.

In the management tool, different meal combinations can be composed by selecting individual foods from a food data library, which stores glycemic effect, digestive speeds and amplitudes as a function of carbohydrate content. The food data library is displayed as food choices 71. For convenience, portion size and preparation, where applicable, are included with each food choice 71, although portion size and preparation could alternatively be separately specified.

The food choices 71 are open-ended, and one or more food items can be added to a planned meal by pressing the "ADD ITEM" button 72. Glycemic effect data, such as the glycemic index 73 and carbohydrates type and content 74 for a particular food item, are retrieved also from the stored food data library and displayed. A cumulative digestive response curve 75 is generated, as further described below with reference to FIGURES 8A-C. The digestive response curve 75 estimates digestive speed and amplitude for the individual patient, which traces postprandial blood glucose rise, peak, and fall based on the patient's carbohydrate sensitivity. The carbohydrate sensitivity can be expressed as a coefficient or other metric that can be applied to population-based glycemic effect data, such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. _____, pending, the disclosure of which is incorporated by reference. The completion of meal planning is indicated by pressing the "Finished" button 76. Further logical control and display elements are possible.

Constituent Digestive Response

A planned meal must be evaluated to determine the insulin needed to compensate for the estimated postprandial rise in blood glucose. FIGURES 8A-C are graphs 80, 82, 84 showing, by way of example, constituent and cumulative digestive response curves 81, 83, 85 for a hypothetical meal. The x-axes represent time in minutes and the y-axes represent cumulative rise of blood glucose measured in milligrams per deciliter (mg/dL). The amplitude of the curves 81, 83, 85 are patient-dependent, as is the timing of the rise.

In general, food consumption modeling focuses on carbohydrates. Simple sugars, the most basic form of carbohydrate, increase blood glucose rapidly. Conversely, more complex carbohydrates, such as whole grain bread, increase blood glucose more slowly due to the time necessary to break down constituent components. Proteins also raise blood glucose slowly, as they must first be broken down into amino acids before being converted into glucose. Fats, which include triglycerides and cholesterol, delay glucose uptake. Thus, carbohydrates, and not proteins or fats, have the largest and most direct affect on blood glucose. Notwithstanding, all foods that contribute to blood glucose rise, not just carbohydrates, can be included in the model.

Each item of food consumed contributes to the overall carbohydrate content and, thence, postprandial blood glucose rise. Referring first to FIGURE 8A, a graph 80 showing, by way of example, a digestive response curve 81 for postprandial blood glucose rise for a six ounce glass of orange juice is provided. The curve 81 reflects a relatively fast and pronounced rise in blood glucose, which peaks about an hour following consumption. Referring next to FIGURE 8B, a graph 82 showing, by way of example, a digestive response curve 83 for postprandial blood glucose rise for a 16 ounce sirloin steak is provided. The curve 83 reflects a comparatively prolonged and modest rise in blood glucose, which peaks about five-and-a-half hours following consumption.

The type of food and manner of preparation can affect glucose uptake. Orange juice is a beverage that is readily metabolized and absorbed into the blood stream, which results in a rapid and significant rise in blood glucose. The rise, though, is short term. In contrast, steak is primarily protein and the manner of preparation will have little affect on carbohydrate content. The rise in blood glucose is delayed by the protein having to first be broken down into amino acids. The resultant equivalent carbohydrate content also is low, thus resulting in a more attenuated rise in blood glucose. Food items principally containing complex carbohydrates are more affected by manner of preparation. For example, pasta prepared "al dente" is slightly undercooked to render the pasta firm, yet not hard, to the bite. The "al dente" form of preparation can increase digestive time and delay glucose uptake. The form of preparation can also be taken into account in the management tool. Finally, some medications can modify the effect of foods on blood glucose. Other effects on food items, as to type and manner of preparation, also are possible.

Cumulative Digestive Response

Except for the occasional snack item, food is generally consumed as a meal. Items of food consumed in combination during a single sitting, as typical in a meal, can cumulatively or synergistically interact to alter the timing and amplitude of blood glucose rise based on the digestive processes involved and the net change to overall carbohydrate content. Referring finally to FIGURE 8C, a graph 84 showing, by way of example, a cumulative digestive response curve 85 for postprandial blood glucose rise for a meal that combines the six ounce glass of orange juice with the 16 ounce sirloin steak is provided. The cumulative digestive response curve 85 combines the respective constituent digestive response curves 81, 83 and proportionately applies the patient's carbohydrate sensitivity. The cumulative curve has an initial near-term peak, which reflects the short time course and high glucose content of the orange juice, and a delayed long term peak, which reflects the protein-delayed and significantly less-dramatic rise in blood glucose attributable to the sirloin steak. Shortly following consumption of the orange juice and steak, blood glucose rise is dominated by the effects of the orange juice while the steak has little effect. Later, the effects of the orange juice dwindle and the effects of the steak dominate the rise in blood glucose. The effects of both foods are present in-between.

The cumulative digestive response $\vec{r}$ can be determined by taking a summation of the constituent digestive responses over the estimated time course adjusted for synergistic effect:

$$\vec{r} = \sum_{i=1}^{n} \vec{d}_i k \qquad (1)$$

where $\vec{d}_i = \{x_1, x_2, \ldots, x_m\}$, such that there are n constituent digestive response vectors, each normalized to length m, and containing digestive response values x; and k is an adjustment coefficient for synergy, such that $k > 0$ . The last element of each constituent digestive response vector is repeated to ensure all constituent digestive response vectors are of the same length. Other cumulative digestive response determinations are possible.

The particular combinations of orange juice and steak have little synergistic effect. The orange juice, as a beverage, metabolizes quickly in the stomach, whereas the steak, as a solid protein, is primarily metabolized in the small intestine following secretion of bile. Other food combinations, though, can synergistically raise or lower the overall carbohydrate level, or accelerate or delay glucose uptake.

Insulin Selection

The selections of insulin and other medications, when applicable, are also key to diabetes management. The patient needs to identify both the type and amount of insulin preparation used and his sensitivity to allow the management tool to generate an insulin response curve. Insulin preparation types are identified by source, formulation, concentration, and brand name, and are generally grouped based on duration of action. FIGURE 9 is a diagram showing, by way of example, a screen shot of a graphical user interface 90 for specifying insulin preparation type for use in the graphical user interface 60 of FIGURE 6. Different types of insulin preparation 91 can be selected and, for ease of use and convenience, are identified by brand name or formulation. Insulin preparations include short-acting insulins, such as lispro or Regular, are used to cover a meal and are frequently administered by insulin pump due to the short time of onset. Intermediate-acting insulins, such as NPH (Neutral Protamine Hagedorn), have 12-18 hour durations, which peak at six to eight hours. Finally, long-acting insulins, such as Ultralente, have 32-36 hour durations to provide a steady flow of insulin. Long-acting insulins are generally supplemented with short-acting insulins taken just before meals. Other types of insulin preparations include insulin glargine, insulin detemir, and insulin preparation mixes, such as "70/30," which is a premixed insulin preparation containing 70% NPH insulin and 30% Regular insulin. In addition, insulin sensitivity 92 and insulin bolus "bump" 93, that is, a single dosing, such as for meal coverage, can be specified, before being factored into the model upon pressing of the "APPLY" button 94. The insulin response curve is adjusted based on the patient's insulin sensitivity. The insulin sensitivity can be expressed as a coefficient or other metric that can be applied to published insulin activity curves, such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. _____, pending, the disclosures of which are incorporated by reference. Further logical control and display elements are possible.

Other Medication Selection

Type 1 diabetics may receive medications in addition to insulin. Each medication should also be identified to allow the management tool to project any effect on glycemic activity. A patient may currently be taking medications in addition to insulin. FIGURE 10 is a diagram showing, by way of example, a screen shot of a graphical user interface 100 for specifying other medications for use in the graphical user interface 60 of FIGURE 6. Different medications 101 can be selected and, for ease of use and convenience, can be identified by generic name, brand name, or formulation. As appropriate, the therapeutic effects, particularly as relating to blood glucose level, and drug interactions of each medication can be factored into the model upon pressing of the "APPLY" button 102. For example, pramlintide acetate, offered under the brand name Symlin, is prescribed to both Type 1 and Type 2 diabetics help lower postprandial blood glucose during the three hours following a meal. Consequently, the blood glucose rise for a Type 1 diabetic would need to be adjusted to reflect the effects of the pramlintide acetate in light of a planned meal and dosed insulin. Further logical control and display elements are possible.

Method

Conventional Type 1 diabetes management relies on patient intuition and experiential awareness of insulin sensitivities. Individualized diabetes management can be significantly improved by modeling quantified patient food and insulin sensitivities. FIGURE 11 is a process flow diagram showing a method for actively managing Type 1 diabetes mellitus on a personalized basis 110, in accordance with one embodiment. Active management proceeds as a cycle of repeated operations that are reflective of basic day-to-day diabetes control. Initially, a personal predictive management tool is established (operation 111), which models both food and insulin sensitivities, such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. _____, pending, the disclosures of which are incorporated by reference. Thereafter, a rise in blood glucose is estimated (operation 112) by determining a cumulative digestive response curve based on the patient's food selections, as described above with reference to FIGURES 8A-C. Based on the cumulative digestive response curve, the insulin dosage needed to counteract the rise in blood glucose is determined (operation 113). The dosage can be estimated, for instance, through a graphical display that provides a forecast curve 67 (shown in FIGURE 6), which predicts combined insulin dosing and postprandial blood glucose. Other insulin dosing estimates are possible.

In a further embodiment, the food data library can be refined to add new food items or to revise the food data (operation 114), as further described below respectively with reference to FIGURE 12. In a still further embodiment, the management tool can directly interact with the patient (operation 115), as further described below respectively with reference to FIGURE 13. The active management operations can be repeated as needed.

## Food Data Library Refinement

Both the types of available food items and their accompanying data may change over time. FIGURE 12 is a process flow diagram showing a routine for refining a food data library 120 for use with the method 110 of FIGURE 11. At a minimum, the food data library 121 contains glycemic effect, digestive speeds and amplitudes as a function of carbohydrate content. The data can be obtained from various sources and is integrated into the library 121. For instance, standardized carbohydrate values 122, for instance, glycemic indices or glycemic load, can be retrieved from authoritative sources, such as the University of Toronto, Toronto, Ontario, Canada. Empirical values 123 can be derived by the patient through experiential observations of glycemic effect by a combination of fasting and pre- and postprandial blood glucose testing. Synergistic values 124 of food combinations, perhaps unique to the patient's personal culinary tastes, could similarly be empirically derived. Other food data values 125 and sources of information are possible.

## Patient Interaction

In the course of providing blood glucose management, a more proactive approach can be taken as circumstances provide. FIGURE 13 is a process flow diagram showing a routine for interacting with a patient 130 for use with the method 110 of FIGURE 11. Interaction refers to the undertaking of some action directly with or on behalf of the patient. The interaction can include suggesting opportune times to the patient at which to perform self-testing of blood glucose (operation 132). Such times include both pre- and postprandial times, particularly when blood glucose rise is estimated to peak. Similarly, alerts can be generated (operation 133), for example, warnings of low blood glucose, or reminders provided (operation 134), such as reminding the patient to take his insulin for high glucose levels. Interaction could also include intervening (operation 136), such as notifying a patient's physician or emergency response personnel when a medical emergency arises. Other forms of patient interaction (136) are possible.

## System

Automated Type 1 diabetes management can be provided on a system implemented through a patient-operable device, as described above with reference to FIGURE 3. FIGURE 14 is a block diagram showing for a system for actively managing Type 1 diabetes mellitus on a personalized basis 140, in accordance with one embodiment. At a minimum, the patient-operable device must accommodate user inputs, provide a display capability, and include local storage and external interfacing means.

In one embodiment, the system 140 is implemented as a forecaster application 141 that includes interface 142, analysis 143, and display 144 modules, plus a storage device 147. Other modules and devices are possible.

The interface module 142 accepts user inputs, such as insulin sensitivity 151, carbohydrate sensitivity 152, patient-specific characteristics 153, and food selections 154. Other inputs, both user-originated and from other sources, are possible. In addition, in a further embodiment, the interface module 142 facilitates direct interconnection with external devices, such as a blood or interstitial glucose monitor, or centralized server (not shown). The interface module 142 can also provide wired or wireless access for communication over a private or public data network, such as the Internet. Other types of interface functionality are possible.

The analysis module 143 includes blood glucose estimator 145 and insulin estimator 146 submodules. The blood glucose estimator submodule 145 forms a personal digestive response curve 148, which is determined from data in the food data library 150 for the food selections 155. The personal digestive response curve 148 can be determined using glycemic effect, digestive speeds and amplitudes as a function of the carbohydrate sensitivity 152. Similarly, the insulin estimator 146 forms an insulin activity curve 149 using, for instance, a population-based insulin activity curve proportionately adjusted by the insulin sensitivity 153. The personal digestive response curve 148 and insulin activity curve 149 are used by the analysis module 143 to generate an estimate 156 of blood glucose rise 157 and insulin required 158. Other analytical functions are possible.

Finally, the display module 144 generates a graphical user interface 155, through which the user can interact with the forecaster 151. Suggestions for blood glucose self-testing times, alerts, and reminders are provided via the display module 144, which can also generate an intervention on behalf of the patient. The user interface 155 and its functionality are described above with reference to FIGURE 4.

While the invention has been particularly shown and described as referenced to the embodiments thereof, those skilled in the art will understand that the foregoing and other changes in form and detail may be made therein without departing from the spirit and scope.

## Claims

1. A system (140) for actively managing Type 1 diabetes mellitus on a personalized basis, comprising:

    a database (147) comprising models of glycemic effect (122) for a Type 1 diabetic patient (21) for both insulin time course (51) and digestive response (41); and
    a forecaster module (143), comprising:

        a blood glucose estimator module (145) configured to estimate a rise in postprandial blood glucose (157) through food ingestion of a planned meal (71) in proportion to the digestive response model (40); and
        an insulin estimator module (146) configured to determine an amount of insulin (158) necessary and timing of delivery (156) to mediate transport of blood glucose into cells in proportion to the postprandial blood glucose rise (157) through the insulin time course model (50).

2. A system (140) according to Claim 1, further comprising:

    a library (120) of digestive responses for foods, which include rises in blood glucose particular to the patient (21), wherein the digestive responses for the foods in the planned meal (71) are aggregated over overlapping time courses as the digestive response model (40).

3. A system (140) according to Claim 2, wherein the library (120) is maintained as glycemic indices (122), and the glycemic indices for the foods (123) in the planned meal (71) are apportioned as glycemic loads based on portion size.

4. A system (140) according to Claim 1, further comprising:

    a refinement module configured to refine the digestive response model (40) through at least one of preprandial and postprandial blood glucose testing.

5. A system (140) according to Claim 1, further comprising:

    a model of glycemic effect for a medication other than insulin (158), wherein a physiological effect on the postprandial blood glucose due to dosing of the medication is determined over a time course of the insulin (158).

6. A system (140) according to Claim 1, wherein the models are expressed as coefficients respectively applied to a population-based insulin time course (51) and empirically-derived digestive response (123).

7. A system (140) according to Claim 1, further comprising:

    a display module configured to interact directly with the patient (21), comprising one or more of:

        a suggestion module configured to suggest times for self-testing blood glucose;
        an alert module configured to generate alerts regarding blood glucose;
        a reminder module configured to provide reminders regarding insulin (158); and
        an intervention module configured to intervene through communication with a caregiver on behalf of the patient (21).

8. A method (30) for actively managing Type 1 diabetes mellitus on a personalized basis, comprising:

    establishing models of glycemic effect for a Type 1 diabetic patient (21) for both insulin time course (51) and digestive response (41);
    estimating a rise in postprandial blood glucose (157) through food ingestion of a planned meal (71) in proportion to the digestive response model (40); and
determining an amount of insulin (158) necessary and timing of delivery (156) to mediate transport of blood glucose into cells in proportion to the postprandial blood glucose rise (157) through the insulin time course model (50).

**9.** A method (30) according to Claim 8, further comprising:

referencing a library (120) of digestive responses for foods, which include rises in blood glucose particular to the patient (21); and
aggregating the digestive responses for the foods in the planned meal (71) over overlapping time courses as the digestive response model (40).

**10.** A method (30) according to Claim 9, further comprising:

maintaining the library (120) as glycemic indices (122); and
apportioning the glycemic indices for the foods (123) in the planned meal (71) as glycemic loads based on portion size.

**11.** A method (30) according to Claim 8, further comprising:

refining the digestive response model (40) through at least one of preprandial and postprandial blood glucose testing.

**12.** A method (30) according to Claim 8, further comprising:

establishing a model of glycemic effect for a medication other than insulin (158); and
determining a physiological effect on the postprandial blood glucose due to dosing of the medication over a time course of the insulin (158).

**13.** A method (30) according to Claim 8, further comprising:

expressing the models as coefficients respectively applied to a population-based insulin time course (51) and empirically-derived digestive response (123).

**14.** A method (30) according to Claim 8, further comprising:

interacting directly with the patient (21), comprising one or more of:

suggesting times for self-testing blood glucose;
generating alerts regarding blood glucose;
providing reminders regarding insulin (158); and
intervening through communication with a caregiver on behalf of the patient (21).

Fig. 1 (prior art).

10

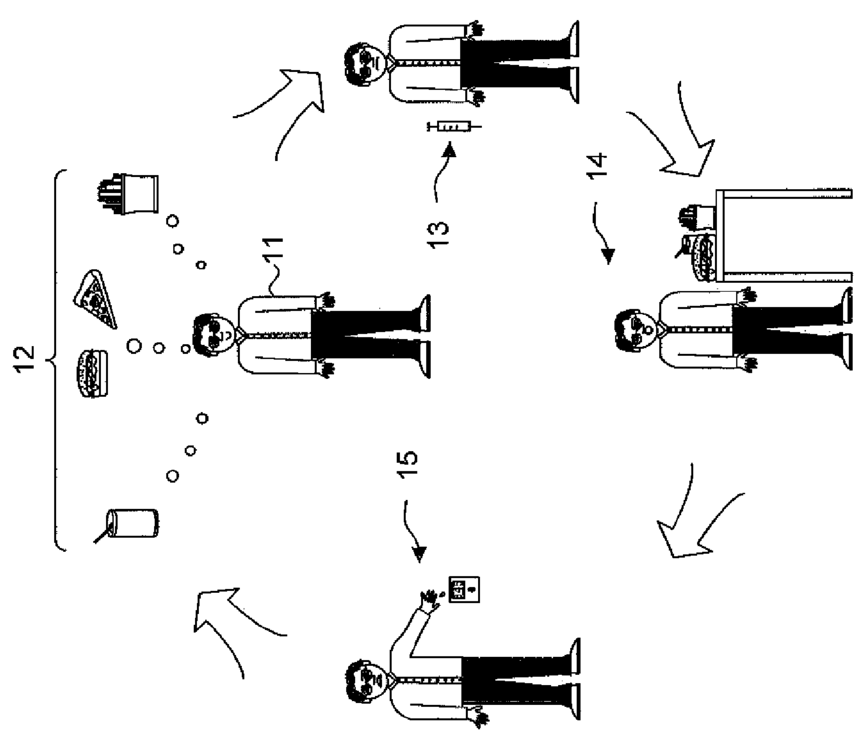


Fig. 2.

20

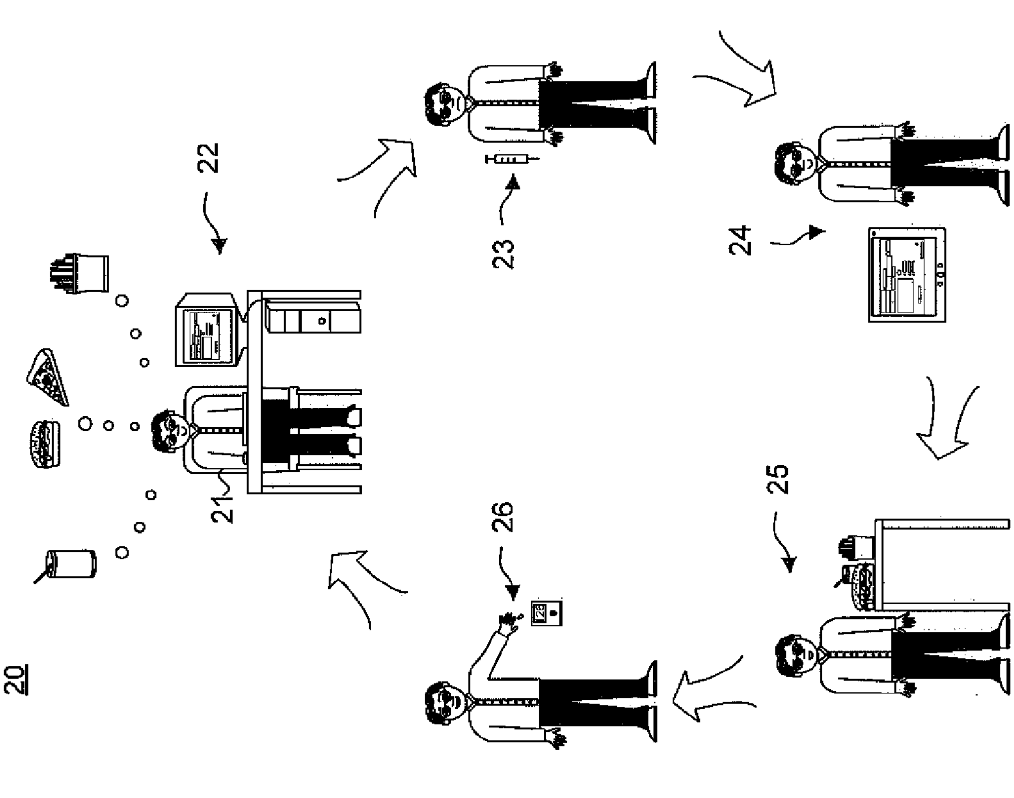

Fig. 3.

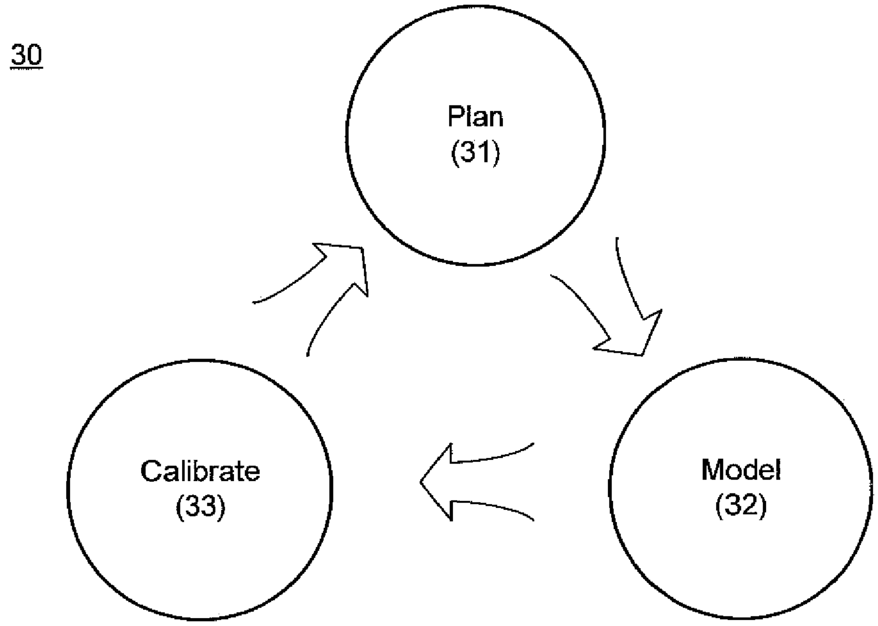
30
Plan
(31)
Model
(32)
Calibrate
(33)

Fig. 4.

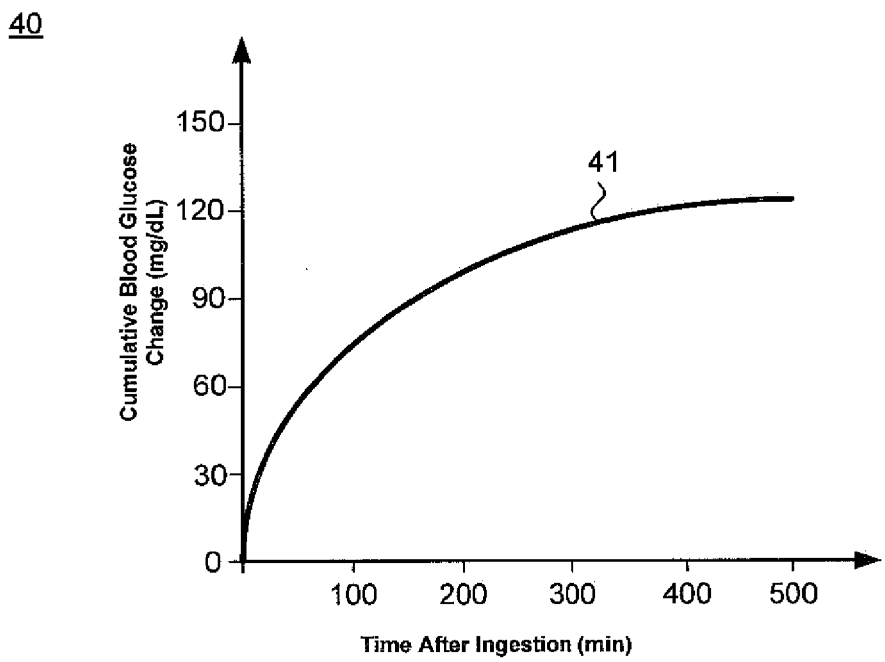
40
Cumulative Blood Glucose Change (mg/dL)
150
120
90
60
30
0
41
100
200
300
400
500
Time After Ingestion (min)

Fig. 5.

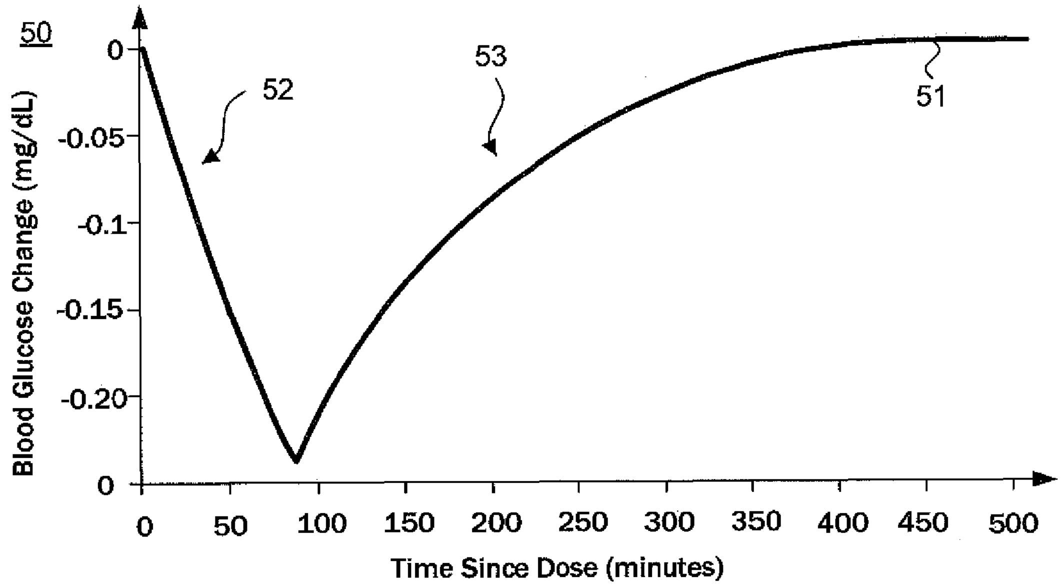
50
0
-0.05
-0.1
-0.15
-0.20
0
Blood Glucose Change (mg/dL)
52
53
51
0
50
100
150
200
250
300
350
400
450
500
Time Since Dose (minutes)

Fig. 6.

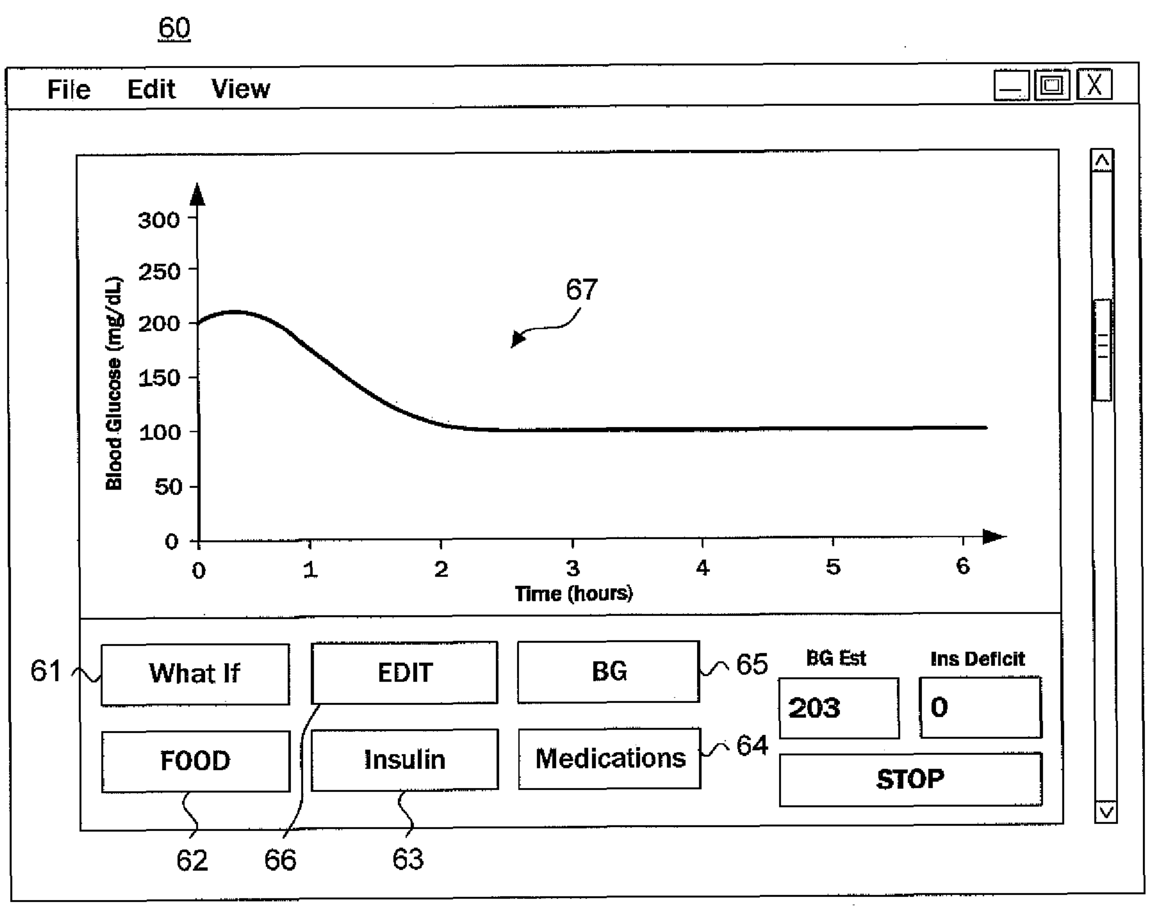
60
File
Edit
View
300
250
200
150
100
50
0
Blood Glucose (mg/dL)
67
0
1
2
3
4
5
6
Time (hours)
61
What If
EDIT
BG
65
BG Est
Ins Deficit
203
0
FOOD
Insulin
Medications
64
STOP
62
66
63

# Fig. 7.

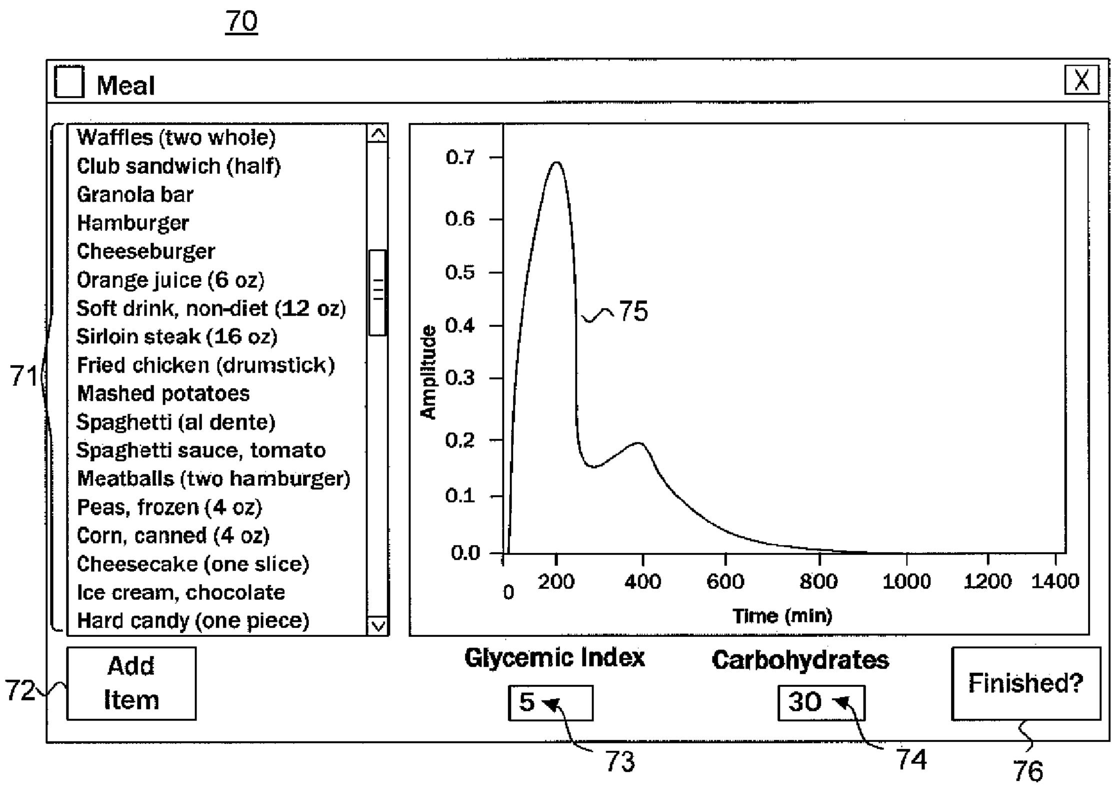


# Fig. 8A.

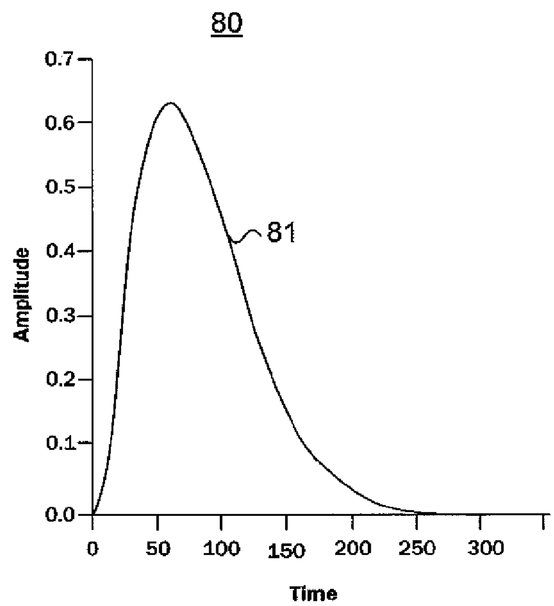


# Fig. 8B.

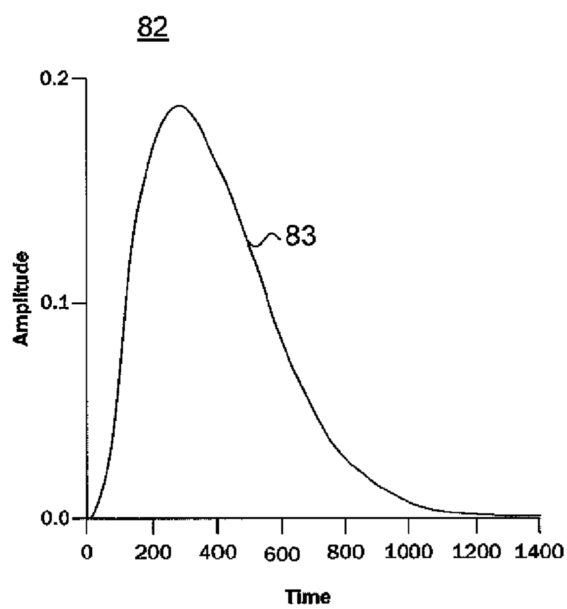

Fig. 8C.

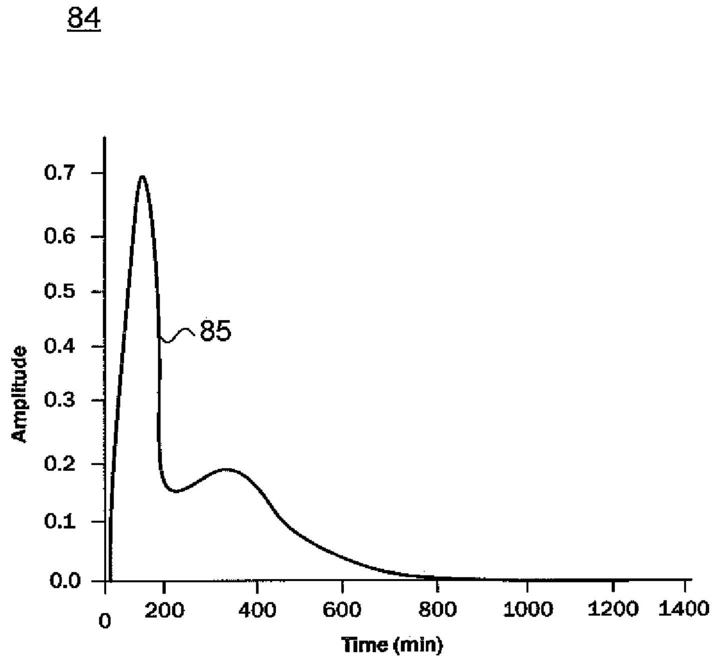


Fig. 9.

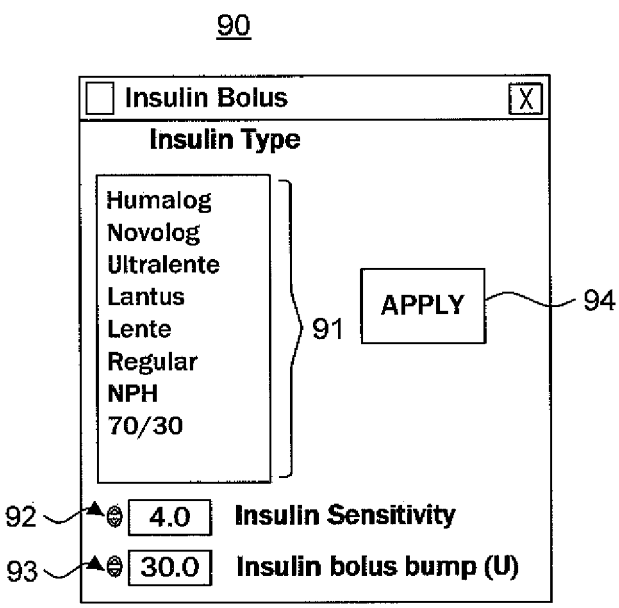


Fig. 10.

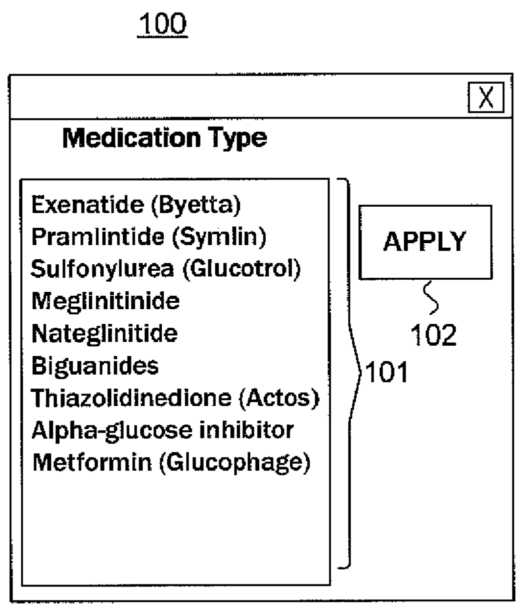

Fig. 11.

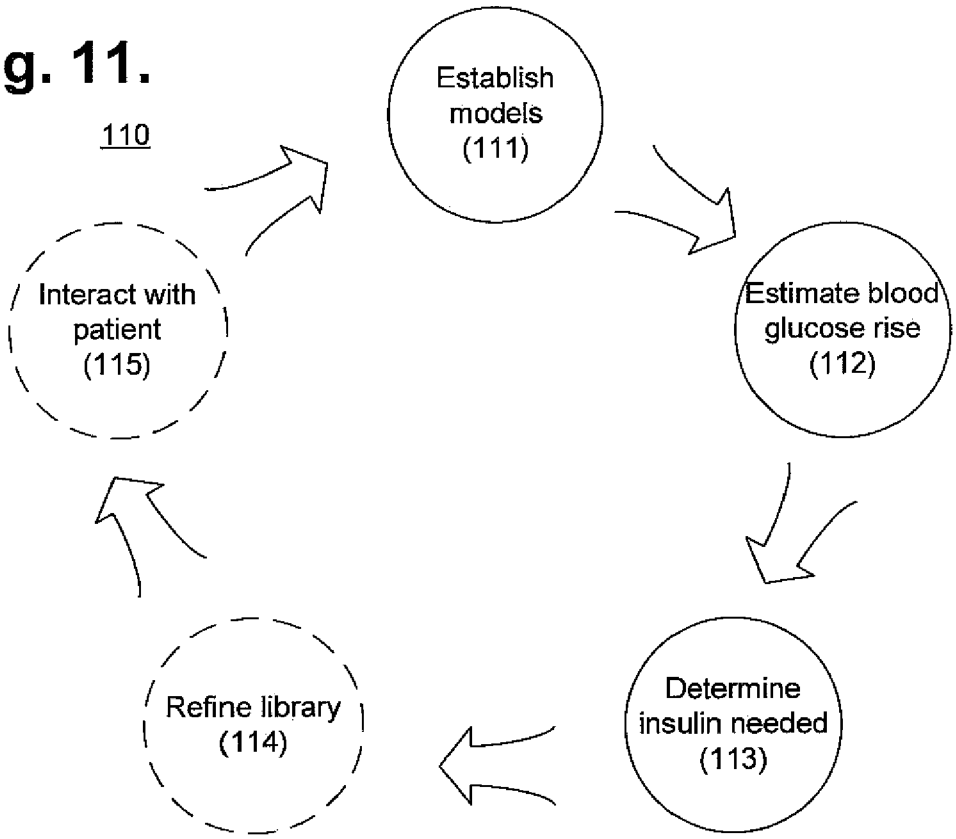
110
Establish models (111)
Estimate blood glucose rise (112)
Determine insulin needed (113)
Refine library (114)
Interact with patient (115)

Fig. 12.

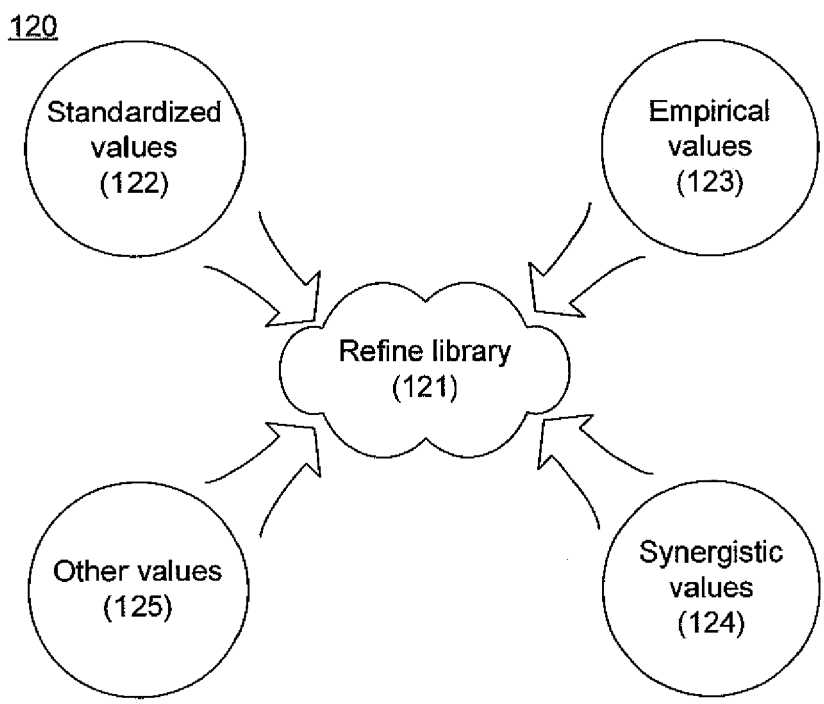
120
Standardized values (122)
Empirical values (123)
Refine library (121)
Other values (125)
Synergistic values (124)

Fig. 13.

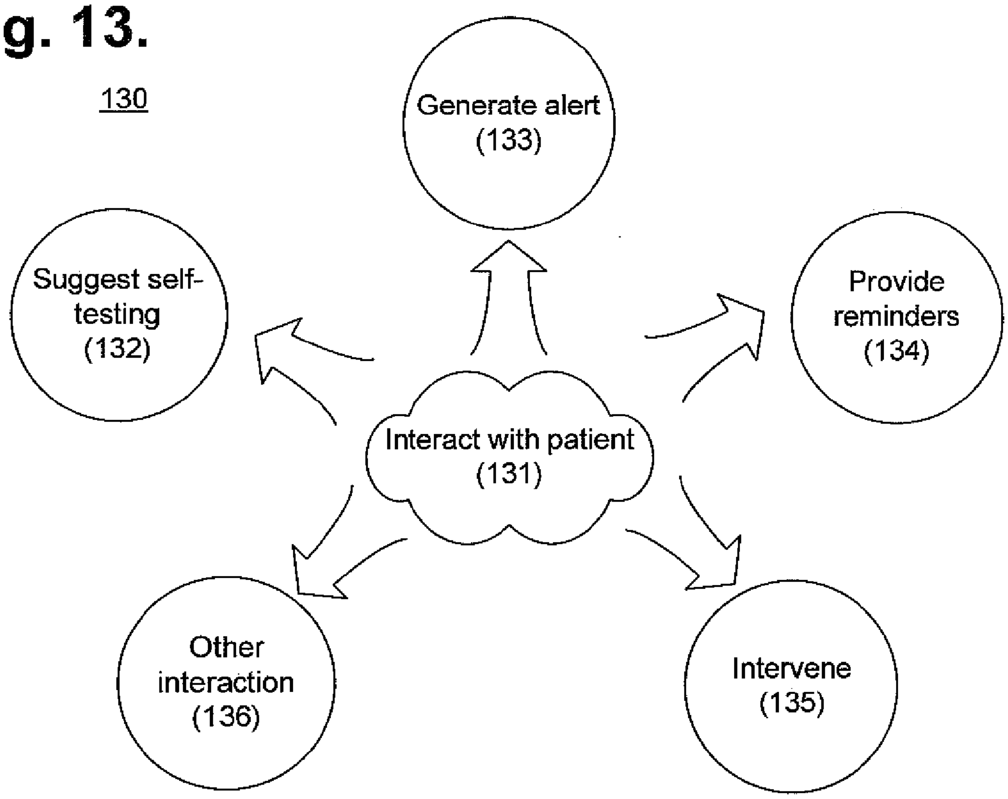
130
Generate alert
(133)
Suggest self-testing
(132)
Provide reminders
(134)
Interact with patient
(131)
Other interaction
(136)
Intervene
(135)

Fig. 14.

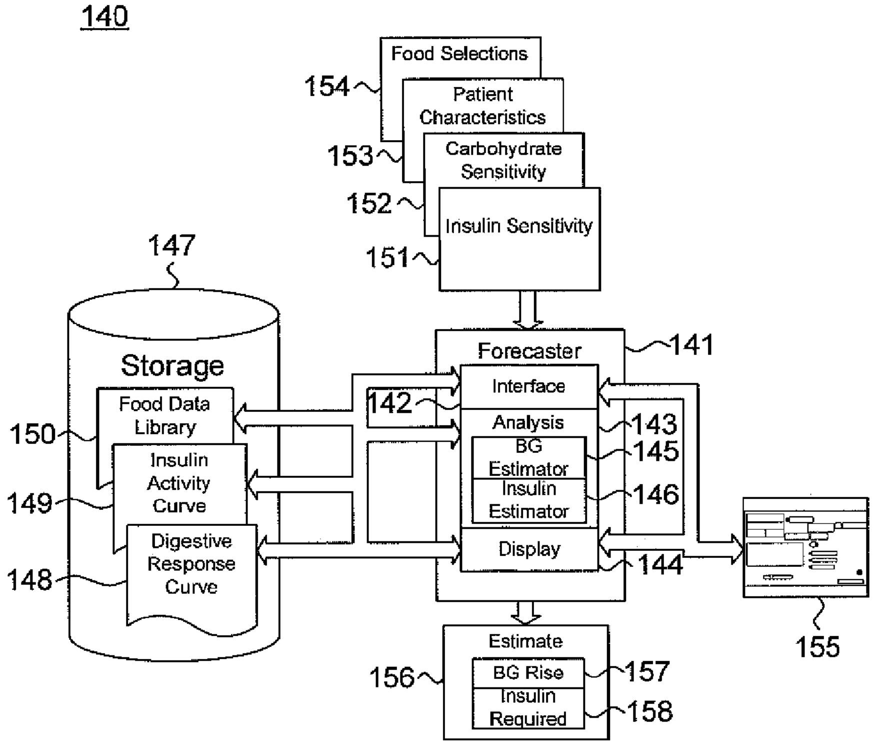
140
Food Selections
154
Patient Characteristics
153
Carbohydrate Sensitivity
152
Insulin Sensitivity
151
147
Storage
Food Data Library
150
Insulin Activity Curve
149
Digestive Response Curve
148
Forecaster
141
Interface
142
Analysis
143
BG Estimator
145
Insulin Estimator
146
Display
144
155
Estimate
156
BG Rise
157
Insulin Required
158

Europäisches Patentamt
European Patent Office
Office européen des brevets

# EUROPEAN SEARCH REPORT

Application Number
EP 10 15 2574

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2003/208113 A1 (MAULT JAMES R [US] ET AL) 6 November 2003 (2003-11-06)<br>* abstract *<br>* paragraphs [0119] - [0134], [ 159], [ 181] - [0188] *<br>----- | 1-14 | INV.<br>G06F19/00 |
| Y | US 6 925 393 B1 (KALATZ BRIT [DE] ET AL) 2 August 2005 (2005-08-02)<br>* the whole document *<br>----- | 1-14 | |

TECHNICAL FIELDS SEARCHED (IPC)

G06F

The present search report has been drawn up for all claims

| Place of search | Date of completion of the search | Examiner |
|---|---|---|
| Munich | 19 May 2010 | Chabros, Cezary |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO.

EP 10 15 2574

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2010

| Patent document cited in search report | Publication date | Patent family member(s) | Publication date |
|---|---|---|---|
| US 2003208113 A1 | 06-11-2003 | NONE | |
| US 6925393 B1 | 02-08-2005 | DE 10057215 A1 | 23-05-2001 |
| | | EP 1102194 A2 | 23-05-2001 |
| | | JP 3594897 B2 | 02-12-2004 |
| | | JP 2001204817 A | 31-07-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6168563 B, Brown
- US 6024699 A, Surwit